# EUROPEAN PATENT SPECIFICATION

(11) **EP 2 728 200 B1**
(45) Date of publication and mention of the grant of the patent: **13.01.2016**
(21) Application number: 13186631.1
(22) Date of filing: 30.09.2013
(51) Int. Cl.: F04F 5/54, A61M 1/00, A61L 2/18, A61L 2/26

(54) **Device for recovering air from an aspirator and system comprising such a device**
Vorrichtung zur Rückgewinnung von Luft aus einem Aspirator und System mit solch einer Vorrichtung
Dispositif de récupération de l'air d'un aspirateur et système comprenant un tel dispositif

(30) Priority: 31.10.2012 IT PR20120072
(43) Date of publication of application: 07.05.2014
(73) Proprietor: CA-MI Societa' a Responsabilita' Limitata, 43013 Langhirano (PR) (IT)
(72) Inventor: Visotti, Andrea, 43013 LANGHIRANO (PR) (IT)
(74) Representative: Gotra, Stefano

(56) References cited:
- WO-A1-2007/108021
- CN-U- 2 050 763
- US-A- 6 003 787
- US-A- 6 043 287
- US-A1- 2004 050 877
- US-A1- 2004 059 284

## Description

The present invention relates to a device for recovering air from an aspirator.

Also part of the invention is a combined system for aspiration and sterilisation that uses the above device, which can recover the air from the aspirator in order to use it in another way.

In particular, the device constituting the subject matter of the present invention can be used with surgical or dental aspirators or with machines for autoclaves which, by creating a vacuum in a closed volume, aspirating air from that volume to introduce it into the external environment, can in fact be considered as aspirators.

The surgical or dental aspirators of the known art are exclusively used for the aspiration of liquids or other organic fluids coming from patients.

All aspirators belonging to the different classes (portable surgical, high flow, fixed in operating theatres, on trolleys in operating theatres, for emergencies built into a special body or portable) operate through an electric motor connected to a pump, which has two outlets.

The first outlet aspirates air during the descent stage of the piston; the second outlet expels air during the ascent stage of the piston.

The first outlet is therefore connected to the aspirator and actively performs the function of aspirating liquids, air or organic fluids.

The second outlet, on the other hand, only emits compressed air, diffusing it into the external environment.

The applicant has found that this second outlet, which is not currently used, could be actively exploited for useful purposes connected with the activity being performed with the aspirator to which the pump is connected. From US 2004/050877 A1 an apparatus for sanitising/disinfecting through the nebulisation of relevant substances through a container of pressurised gas is known .

CN 2050763U and US 2004/0059284 A1 show two different devices adapted to inject and aspirate liquids. In actual fact the first document does not show any reversibility, whereas the second document even envisages the need to used two pumps.

Further devices adapted to spray disinfectants or insecticides are known by US 6043287A and US 6003787.

An object of the present invention is to overcome the drawbacks of the known art.

In particular, an object of the present invention is to provide a device able to recover the air from an aspirator, in particular the air at the outlet of a pump connected to an aspirator (up to now used in surgical aspirators of the known art), and use it actively for purposes connected with medical activities.

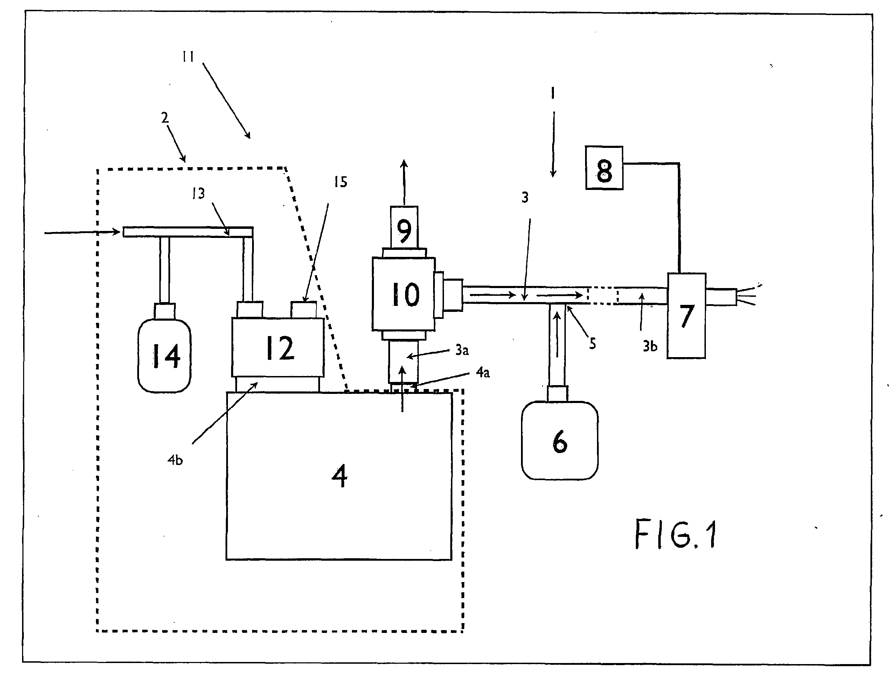

Further characteristics and advantages of the present invention will more fully emerge from the non-limiting description of a preferred but not exclusive embodiment of a device for the recovery of air from an aspirator and a combined system that uses such a device, as illustrated in the accompanying drawing as Figure 1, which illustrates a schematic view of a combined system for aspiration and sterilisation comprising a device for recovering air from an aspirator in accordance with the present invention. The reference number 1 has been used to generally indicate a device for recovering air from an aspirator, for example a surgical aspirator, indicated schematically with the number 2.

This device 1 comprises a conduit 3 for transporting a fluid, defining an inlet 3a and an outlet 3b.

The inlet 3a of the conduit 3 can be connected to an outlet 4a of compressed air of a pump 4 of the aspirator 2.

The pump 4 therefore comprises an inlet 4b, which aspirates air during the descent stage of the piston of the pump 4, and the air outlet 4a that expels air during the ascent stage of the piston, creating compressed air.

The conduit 3 is in fluid communication with a tank 6 containing a sterilising agent.

The connection between the conduit 3 and the tank 6 takes place at a collection section 5, through which the sterilising agent is introduced into the conduit 3.

In particular, this connection between the tank 6 containing the sterilising agent and the conduit 3 takes place in correspondence with a mixing zone in which the mixture of the sterilising agent with the compressed air coming from the pump 4 takes place due to the Venturi effect.

To obtain the Venturi effect, the conduit 3 is shaped appropriately and therefore has a cylindrical section with a decreasing diameter from the inlet 3a to the outlet 3b.

The device 1 also comprises a dispensing terminal 7 of the mixture of compressed air and sterilising agent connected to the outlet 3b of the conduit 3.

The dispensing terminal 7 may be a gun equipped with a nozzle or a diffuser.

In the latter case, the diffuser may be fixed or assembled, for example, on the aspirator or on other apparatuses such as ambulances or dentists' chairs.

The tank 6 containing the sterilising agent can be refillable or interchangeable as required with a full one.

Other alternative embodiments, not illustrated, envisage the tank 6 containing the sterilising agent is positioned in proximity to the pump 4, therefore near the inlet 3a of the conduit 3, or with a joint under the dispensing terminal 7, therefore in proximity to the inlet 3b of the conduit 3 or even positioned far from the conduit 3 and connected thereto with a pipe.

According to a further embodiment, the pump and tank may be contained in the same casing or body of the surgical aspirator.

The presence of a control element 8 of the dispensing of the mixture of compressed air and sterilising agent is also envisaged.

This control element 8 is, for example, a pushbutton panel located on the device 1 or directly on the dispensing terminal 7, or even positioned at a distance and such as to exercise a remote control effect.

The control element 8 can advantageously also regulate the flow rate of the dispensing of the mixture of compressed air and sterilising agent.

In the event that it is not necessary to sanitise surfaces or sterilise instruments for medical use, the compressed air leaving the pump can be directly discharged into the external environment.

In order to do so, the device 1 may comprise an exhaust conduit 9 and a relative switch 10, which closes the inlet to the conduit 3 and selectively sends the compressed air directly into the external environment, through the exhaust conduit 9, preventing it from being mixed with the sterilising agent.

This switch may be activated by the control element 8.

The device 1 constituting the subject matter of the present invention is advantageously usable with aspirators of the known art already on the market or already used in hospitals or first aid units. In fact, it is sufficient to connect it to the outlet 4a of the aspirator pump 4. It can also be integrated within a combined system 11 for aspiration and sterilisation. This system 11 comprises a pump 4, an aspirator 2 connected to an inlet 4b of the pump 4 and the device 1 described above connected to the outlet 4a of the pump.

Advantageously, the aspirator 2 may be a surgical aspirator of the fixed, portable, trolley-mounted or emergency type, or a fixed or trolley-mounted autoclave.

In fact, the autoclave aspirates air to create a vacuum inside a closed volume, and hence emits the air taken from the closed volume directly into the external environment.

Therefore, an autoclave can also be considered an aspirator and therefore can be coupled with the device constituting the subject matter of the present invention.

This device may be preferably supplied with alternating current at any voltage, direct current or a battery.

The invention thus attains the set aim.

In fact, the use of the second outlet to create the nebulisation of a sterilising agent enables a combined use of an aspirator to be obtained using the same pump as the aspirator.

The operating principle is based on the Venturi tube: The compressed air leaving the pump passes into the cylindrical conduit which, having a decreasing diameter, increases its speed.

The increase in speed creates a decrease in pressure within the cylindrical conduit with the consequent aspiration of the sterilising agent.

Within the conduit, which is in fluid communication with the tank, the mixture of the two fluids is created with consequent nebulisation.

According to one embodiment, a further switch 12 is envisaged on the inlet 4b of the pump 4. Said further switch 12 has the function of switching the inlet of air into the pump 4 between an inlet 13 (where the air is taken from a vessel 14 being part of the aspirator 2) and an inlet 15 (where the air is taken from within the casing of the aspirator 2). When the air is taken from the inlet 13 the operation is that of a surgical aspirator, whereas when the air is taken from the inlet 15 the operation is that of a sanitiser.

A terminal, like a handpiece, connected to the outlet of the conduit, enables the sterilising agent to be diffused, in a targeted manner and if necessary, in a nebulised form, hence enabling any surface or instrument to be sanitised with the same system as that for the aspiration, by simply exploiting the second outlet of the pump.

Therefore the device can recover the air from an aspirator, in particular the compressed air from the pump of a surgical aspirator, to nebulise liquid substances for medical use.

The same principle can also be used with a machine for autoclaves, used to create a vacuum, which has a two-way pump inside it.

## Claims

1. A device for recovering air from an aspirator (2) comprising a conduit (3) for transporting a mixture of compressed air and a sterilising agent, a dispensing terminal (7) of said mixture connected to an outlet (3b) of said conduit (3) and a tank (6) containing a sterilising agent in fluid communication with said conduit (3) through a collection section of said sterilising agent;
**characterizing in that** said conduit (3) comprises
an inlet (3a) connectable to an outlet (4a) of compressed air of a pump (4) of the aspirator;
a switch (10) which selectively sends the compressed air towards a mixing zone within said conduit (3) or towards an exhaust conduit (9) through which the compressed air is transferred directly into the external environment.

2. The device according to claim 1, wherein said conduit (3) has a cylindrical section and has a decreasing diameter from the inlet (3a) to the outlet (3b).

3. The device according to the previous claim, wherein the connection between the tank (6) containing the sterilising agent and the conduit (3) takes place in correspondence with a mixing zone in which said sterilising agent is collected from said tank (6) due to the Venturi effect.

4. The device according to one of the previous claims, wherein said dispensing terminal (7) is a diffuser or a pistol equipped with a nozzle.

5. The device according to one of the previous claims, wherein said tank (6) is refillable.

6. The device according to one of the previous claims, wherein said tank (6) is interchangeable.

7. The device according to one of the previous claims in which there is a further switch (12) at the inlet of the pump (4) to switch the inlet of air into the pump (4) between an inlet (13) (where the air is taken from a vessel (14) being part of the aspirator (2) and an inlet (15) (where the air is taken from within the casing of the aspirator (2).

8. The device according to one of the previous claims, comprising a control element (8) of the dispensing of the mixture of compressed air and sterilising agent.

9. A combined system for aspiration and sterilisation comprising a pump (4), an aspirator (2) connected to an inlet (4a) of said pump (4) and a device (1) for recovering air coming from said aspirator (2) according to any of claims 1 to 7.

10. The system according to claim 9, wherein said aspirator (2) is a surgical aspirator or an autoclave.

## Patentansprüche

1. Vorrichtung zur Rückgewinnung von Luft aus einem Aspirator (2), umfassend eine Leitung (3), um ein Gemisch aus Druckluft und eines Sterilisationsmittels zu transportieren, ein Ausgabeendstück (7) dieses Gemischs, verbunden mit einem Auslass (3b) der Leitung (3), und einen Tank (6), enthaltend ein Sterilisationsmittel in Fluidkommunikation mit der Leitung (3) durch einen Sammelbereich des Sterilisationsmittels,
**dadurch gekennzeichnet, dass** die Leitung (3) umfasst einen Einlass (3b), der mit einem Auslass (4a) von Druckluft einer Pumpe (4) des Aspirators verbunden werden kann;
einen Schalter (10), der die Druckluft selektiv zu einer Mischzone in der Leitung (3) oder zu einer Abluftleitung (9), durch die die Druckluft direkt in die Außenumgebung transferiert wird, leitet.

2. Vorrichtung nach Anspruch 1, wobei die Leitung (3) einen zylindrischen Bereich aufweist und vom Einlass (3a) zum Auslass (3b) einen abnehmenden Durchmesser besitzt.

3. Vorrichtung nach dem vorhergehenden Anspruch, wobei die Verbindung zwischen dem Tank (6), der das Sterilisationsmittel enthält, und der Leitung (3) an einer Mischzone stattfindet, in der das Sterilisationsmittel aufgrund des Venturi-Effekts vom Tank (6) gesammelt wird.

4. Vorrichtung nach einem der vorhergehenden Ansprüche, wobei das Ausgabeendstück (7) ein Verteiler oder eine Pistole, ausgestattet mit einer Düse, ist.

5. Vorrichtung nach einem der vorhergehenden Ansprüche, wobei der Tank (6) auffüllbar ist.

6. Vorrichtung nach einem der vorhergehenden Ansprüche, wobei der Tank (6) austauschbar ist.

7. Vorrichtung nach einem der vorhergehenden Ansprüche, wobei ein weiterer Schalter (12) am Einlass der Pumpe (4) bereitgestellt ist, um den Einlass von Luft in die Pumpe (4) zwischen einem Einlass (13), in dem die Luft aus einem Behälter (14) entnommen wird, der Teil des Aspirators (2) ist, und einem Einlass (15), in dem die Luft aus dem Bereich innerhalb des Gehäuses des Aspirators (2) entnommen wird, umschaltet.

8. Vorrichtung nach einem der vorhergehenden Ansprüche, umfassend ein Kontrollelement (8) des Ausgebens des Druckluft-Sterilisationsmittel-Gemischs.

9. Kombiniertes System zum Absaugen und Sterilisieren, umfassend eine Pumpe (4), einen Aspirator (2), verbunden mit einem Einlass (4a) der Pumpe (4), und eine Vorrichtung (1) zur Rückgewinnung der aus dem Aspirator (2) eingeleiteten Luft nach einem der Ansprüche 1 bis 7.

10. System nach Anspruch 9, wobei der Aspirator (2) ein chirurgischer Aspirator oder ein Autoklav ist.

## Revendications

1. Dispositif de récupération de l'air d'un aspirateur (2) comprenant un conduit (3) destiné à transporter un mélange d'air comprimé et d'un agent de stérilisation, un terminal de distribution (7) dudit mélange relié à une sortie (3b) dudit conduit (3) et un réservoir (6) contenant un agent de stérilisation en communication fluidique avec ledit conduit (3) à travers une section de collecte dudit agent de stérilisation ;
**caractérisé en ce que** ledit conduit (3) comprend une entrée (3a) pouvant être reliée à une sortie (4a) d'air comprimé d'une pompe (4) de l'aspirateur ;
un interrupteur (10) qui envoie de façon sélective l'air comprimé vers une zone de mélange à l'intérieur dudit conduit (3) ou vers un conduit d'évacuation (9) à travers lequel l'air comprimé est transféré directement dans l'environnement extérieur.

2. Dispositif selon la revendication 1, dans lequel ledit conduit (3) possède une section cylindrique et possède un diamètre décroissant de l'entrée (3a) vers la sortie (3b).

3. Dispositif selon la revendication précédente, dans lequel la liaison entre le réservoir (6) contenant l'agent de stérilisation et le conduit (3) a lieu en correspondance d'une zone de mélange dans laquelle ledit agent de stérilisation est collecté à partir dudit réservoir (6) suite à l'effet Venturi.

4. Dispositif selon l'une des revendications précédentes, dans lequel ledit terminal de distribution (7) est un diffuseur ou un pistolet équipé d'une buse.

5. Dispositif selon l'une des revendications précédentes, dans lequel ledit réservoir (6) est rechargeable.

6. Dispositif selon l'une des revendications précédentes, dans lequel ledit réservoir (6) est interchangeable.

7. Dispositif selon l'une des revendications précédentes, dans lequel se trouve un interrupteur supplémentaire (12) situé au niveau de l'entrée de la pompe (4) pour faire basculer l'entrée d'air dans la pompe (4) entre une entrée (13), où l'air est soutiré d'un récipient (14) faisant partie de l'aspirateur (2), et une entrée (15), où l'air est soutiré de l'intérieur du boîtier de l'aspirateur (2).

8. Dispositif selon l'une des revendications précédentes, comprenant un élément de commande (8) de la distribution du mélange d'air comprimé et de l'agent de stérilisation.

9. Système combiné destiné à l'aspiration et à la stérilisation comprenant une pompe (4), un aspirateur (2) relié à une entrée (4a) de ladite pompe (4) et un dispositif (1) destiné à récupérer l'air provenant dudit aspirateur (2) selon l'une quelconque des revendications de 1 à 7.

10. Système selon la revendication 9, dans lequel ledit aspirateur (2) est un aspirateur chirurgical ou un autoclave.
